# EUROPEAN PATENT SPECIFICATION

(11) **EP 0 575 432 B1**
(45) Date of publication and mention of the grant of the patent: **08.12.2004**
(21) Application number: 92906661.1
(22) Date of filing: 24.02.1992
(51) Int. Cl.: A61K 38/00, A61P 31/14

(54) **MODIFIED PROTEINS AND THEIR USE FOR CONTROLLING VIRAL INFECTIONS**
MODIFIZIERTE PROTEINE UND DEREN VERWENDUNG ZUR KONTROLLE VON VIRUSINFEKTIONEN
PROTEINES MODIFIEES ET LEUR UTILISATION DANS LE CONTROLE D'INFECTIONS VIRALES

(30) Priority: 11.03.1991 NL 9100434
(43) Date of publication of application: 29.12.1993
(73) Proprietor: RIJKSUNIVERSITEIT TE GRONINGEN, 9700 AB Groningen (NL)
(72) Inventor: JANSEN, Robert, Walter, NL-9712 KP Groningen (NL); MEIJER, Dirk, Klaas, Fokke, NL-9724 AN Groningen (NL); MOLEMA, Grietje, NL-9713 JV Groningen (NL); DE CLERCQ, Erik, Désiré, Alice, B-3360 Lovenjoel (BE); PAUWELS, Rudi, Wilfried, Jan, B-1982 Weerde (BE); SCHOLS, Dominique, B-3000 Leuven (BE)
(74) Representative: van Westenbrugge, Andries
(86) International application number: PCT/NL1992/000036
(87) International publication number: WO 1992/015316

(56) References cited:
- EP-A- 0 406 416

## Description

The invention relates to pharmaceutical preparations which are suitable for treating or curing viral infections, including influenza and immunodeficiency diseases such as retrovirus infections, including AIDS and AIDS-related diseases, and which can also be used to inhibit fusion of virus-infected cells with non-infected cells, which preparations contain modified proteins or polypeptides as active substance or substance contributing to the action or as carrier for other substances, which are also active.

The use of glycoproteins as cell-specific carriers for 3'-azido-3'-desoxythymidine (AZT) is disclosed in the publication by Molema, G., Jansen, R.W., Pauwels, R., De Clerq, E., and Meijer, D.K.F. in Biochem. Pharmacol. part 40, No. 12, pp. 2603-2610 (1990). To date AZT is the only registered agent for the treatment of AIDS. AZT blocks the synthesis of viral DNA in the infected cells. Although AIDS is not cured, the agent prolongs life expectancy. Unfortunately, AZT also attacks healthy cells and therefore gives rise to a large number of undesirable side effects. These side effects arise in particular in tissues where a large amount of DNA is produced, for example in bone marrow. According to the abovementioned publication, it is proposed to direct AZT to target cells with the aid of a carrier molecule. According to the abovementioned publication, conjugates of albumin (HSA) and sugars, for example mannose, fucose, galactose and glucose, are prepared and tested for their anti-HIV activity in combination with AZTMP (AZT phosphorylated to the monophosphate form). It is presumed that the carrier molecule releases the active substance once the conjugate of glycoprotein and active substance has been absorbed by the target cell.

The use of sulphated phenyl polymers in preparations for the treatment of retrovirus infections is disclosed in EPA 384.532. Sulphated phenyl polymers mentioned in this publication are sulphated polyphenyl alcohols, sulphated copolymers of (meth)acrylic acid and phenyl alcohol and pharmaceutically acceptable salts thereof. These active substances reduce the cytopathogenicity of HIV-1 in MT-4 cells and the antigen expression of HIV-1 in CEM cells. They are also active against the replication of HIV-2. In addition, the formation of giant cells (multinuclear syncytium cells), generated by HIV-1, and the adsorption of HIV fragments on CD-4 positive cells are inhibited.

The invention relates to pharmaceutical preparations which are suitable for treating or curing viral infections and immuno-deficiency diseases such as retrovirus infections, including AIDS and AIDS-related diseases, and which can also be used to inhibit fusion of virus-infected cells with non-infected cells, which preparations contain modified proteins or polypeptides as active substance or substance contributing to the action or as carrier for other substances, which are also active, which modified proteins or polypeptides have acquired an additional net negative charge by derivatisation of their amino groups and/or other basic functional groups with a reagent which prevents protonation of basic amino acids and/or other basic functional groups or replaces said basic amino acids and/or other basic functional groups by one or more functional groups having a negative charge, the prolongation of the retention time of the protein or polypeptide derivative compared with that of the protein or polypeptide not converted to a derivative being at least 9 minutes, the retention time being determined by means of FPLC on an anion exchange column.

The present invention is based on the finding that a correlation exists between the negative charge of the modified proteins or polypeptides and the antiviral activity. The more negative the active substance, the greater is the antiviral activity, in particular the anti-HIV activity.

EP A1 406.416 discloses an anti-HIV agent which contains as active ingredient a plasma protein modified with a dicarboxylic acid, e.g. maleic anhydride or succinic anhydride, thereby converting amino groups so that their polarity are changed from positive to negative.

As already stated above, the "additional net negative charge" can be quantified on the basis of the retention time. This retention time is determined, for example, in accordance with the following chromatographic method:
- Prepare a solution of substance to be tested in a concentration of 1 mg/ml of a Tris.HCl buffer (0.02 M) having a pH of 7.5 (buffer A).
- Inject 100 µl of the sample into a FPLC system (Fast Protein Liquid Chromatography) from Pharmacia, Woerden, the Netherlands, which is provided with a mono-Q^{R}-HR 5/5 anion exchange column from Pharmacia.
- Elute at a rate of 0.25 ml/min using a gradient of 100% buffer A to 100% buffer B, consisting of buffer A + 1 M NaCl, in 30 minutes.
- Measure the retention time.

The "additional net negative charge" can also be quantified in another way, that is to say on the basis of the theoretical net negative charge. This theoretical value is calculated by multiplying the number of derivatised groups by the change in charge per derivatised group, plus the charge of the starting material. In the case of the negatively charged polypeptides or modified proteins according to the invention, the net negative charge will be lower than -60 and, for example, of the order of magnitude of -60 to -300 and below.

The criterion of the theoretical net negative charge is illustrated with reference to the following examples.

In the compound Suc-HSA (a reaction product of human serum albumin and succinic anhydride) which can be used according to the invention, all 61 positive NH₂ + of lysine have been replaced by negative COO⁻ groups. Thus, there is a change in charge of 2 per lysine. The theoretical charge of this compound is therefore: -15 (from the original albumin or HSA)+(2x -61)=-137. For bovine serum albumin (BSA) the value for the Suc-BSA material would be -140 because the value of the charge on the original BSA is -15.

Suc-HSA, the reaction product of human serum albumin and succinic anhydride which can be used according to the invention, has a theoretical net negative charge of -137 (=-15 + 61 x -2).

Aco-HSA, the reaction product of human serum albumin and aconitic anhydride (anhydride of cis- or trans-prop-1-ene-1,2,3-tri-carboxylic acid) which can be used according to the invention, has a theoretical net negative charge of -198 (= -15 + (61 x -2)).

The negative charge for the very stable reaction product of albumin and propane-1,2,3-tricarboxylic acid anhydride, which is to be preferred, is similar.

Moreover, the prolongation of the retention time compared with that of HSA, determined under the chromatography conditions described, is 13 minutes for Aco-HSA and 11.5 minutes for Suc-HSA.

Moreover, the theoretical charge on the proteins/polypeptides is calculated as the average of the charge on all amino acids present in the molecule (about 600 amino acids in the case of serum albumin).

It is also possible to introduce three or more carboxyl groups per amino group/basic group into the starting materials which can be used according to the present invention. Of course, active materials are then obtained which have an even higher negative charge and a prolongation of the retention time which is even greater than the abovementioned value.

The modified proteins or polypeptides which contain an additional net negative charge and can be used in the pharmaceutical preparations according to the invention are designated "negatively charged polypeptides" in the remainder of this description. Compounds of this type are generally water-soluble and they can exist in ionic form.

The negatively charged polypeptides are preferably poly-amino acids and modified plasma proteins, such as albumin and α-acid glycoproteins ("orosomucoid"). Polypeptides having a molecular weight of the order of magnitude of about 70,000 are suitable. However, the molecular weight can also be appreciably higher or lower; the important factor is that a poly-anionic polypeptide structure is present in the molecule.

It has been found that lysine and histidine are preferred as groups to be derivatised in the negatively charged polypeptides to be used according to the invention. The nitrogen-containing group in lysine is an amino group, whereas the nitrogen-containing group in histidine is regarded as an "other basic functional group". Moreover, for example, polylysine can also be used successfully according to the invention.

Standard chemicals which are capable of introducing an additional net negative charge into the starting material are used to prepare the derivative. Preferably, however, aldehydes, anhydrides, acid chlorides and iso(thio)cyanates are used. It has been found that reagents of this type yield negatively charged polypeptides which provide a pharmaceutical preparation with an outstanding action against the abovementioned disorders and conditions.

The following substances may be mentioned as examples of reagents for preparing derivatives of proteins or polypeptides.
1. Anhydrides
   1.1. Straight-chain anhydrides, which are not part of the invention
      Give, by reaction with amines in proteins, amide bonds which are not protonated at a physiological pH.
      NH₃⁺ → neutral amide.
      (change in charge of -1).
      1.1.1. Symmetrical anhydrides:
         Acetic anhydride 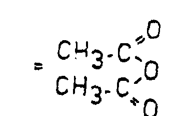 Propionic anhydride 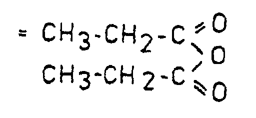 Butyric anhydride 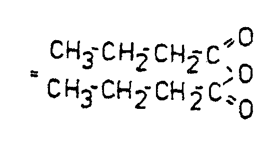
      1.1.2. Asymmetric anhydrides:
         Acetic propionic anhydride 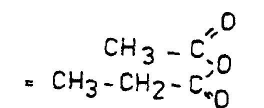 Acetic butyric anhydride 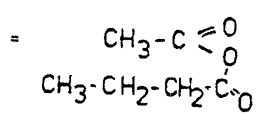
   1.2.1. Cyclic anhydrides, which are not part of the invention
      Give, by reaction with amines in proteins, amide bonds which are not protonated at a physiological pH and in addition they introduce a carboxyl group which is deprotonated at a physiological pH.
      NH₃⁺ → neutral amide + negative carboxyl group.
      (change in charge of -2)
      Succinic anhydride 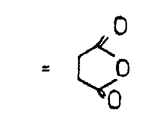 Maleic anhydride 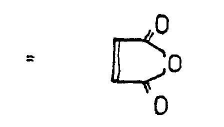 Glutaric anhydride 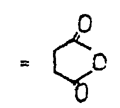 Phthalic anhydride 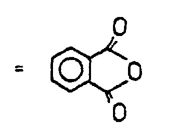
   1.2.2. Cyclic anhydrides having an additional carboxyl group in the ring.

   Give, by reaction with amines in proteins, amide bonds which are not protonated at a physiological pH and in addition they introduce two carboxyl groups which are deprotonated at a physiological pH.
   NH₃⁺ → neutral amide + 2 negative carboxyl groups.
   (change in charge of -3).
   cis- or trans-aconitic anhydride 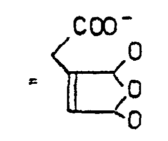 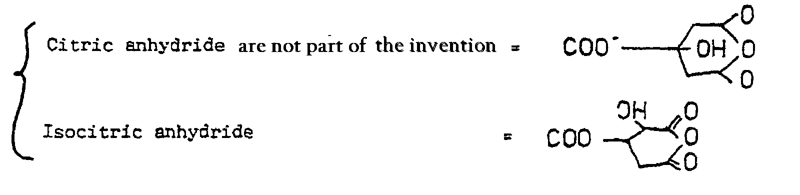
2. Acid halides (in general acid chlorides), which are not part of the invention
   Give, by reaction with amines in proteins, amide bonds which are not protonated at a physiological pH.
   NH₃⁺ → neutral amide.
   (change in charge of -1).
   Acetyl fluorid 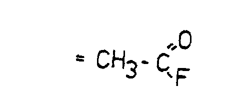 Acetyl chloride 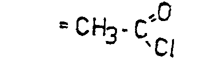 Acetyl bromide 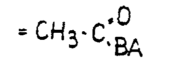 Propionyl chloride 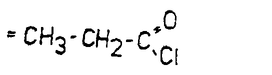 Butyryl chloride 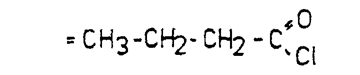 Stearyl chloride 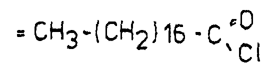 Phosgene (carbonyl chloride) 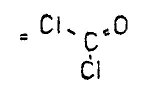 Benzenesulphonyl chloride 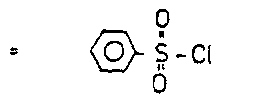 Chlorosulphonic acid 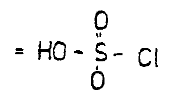
   The last two give a change in charge of -2 because, in addition to the removal of the positive charge from the amino group, a negative charge is introduced on the sulphonyl group.
3. Aldehydes which are not part of the invention.
   Give imines by reaction with amines in proteins.
   NH₃⁺ → neutral imine.
   (change in charge of -1).
   Formaldehyde 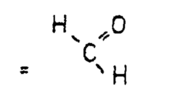 Acetaldehyde 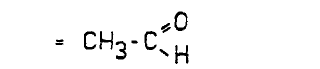 Propionaldehyde 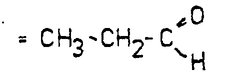 Butyraldehyde 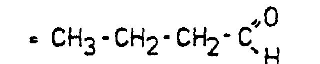 Valeraldehyde 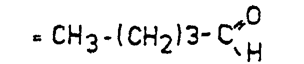 Caproaldehyde 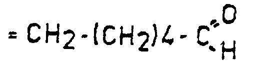
4. Isothiocyanates, which are not part of the invention
   Give, by reaction with amines in proteins, thiocarbamyl bonds which are not protonated at a physiological pH.
   NH₃⁺ → neutral thiocarbamyl.
   (change in charge of -1).
   Benzene isothiocyanate 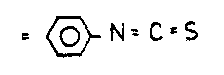 Fluorescein isothiocyanate 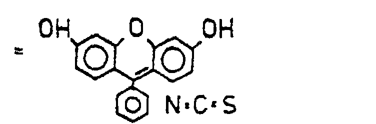 Rhodamine isothiocyanate 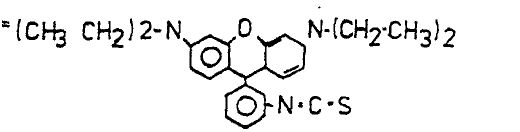 para-Isothiocyanatophenyl sugar derivatives 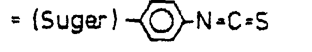
5. Isocyanates, which are not part of the invention
   Give, by reaction with amines in proteins, urea bonds which are not protonated at a physiological pH.
   NH₃⁺ → neutral urea.
   (change in charge of -1).
   Benzene isocyanate

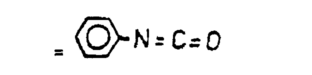

The abovementioned reagents for obtaining poly-anionic proteins or polypeptides are far from complete and give only a few examples from the various groups.

In the case of all reagents, additional anionic functional groups, such as phosphates, sulphates and carboxyl groups, can, where possible, be incorporated in the chain in such a way that additional negative charges are introduced.

Because the additional net negative charge on the polypeptides which are used according to the invention is very important for the action, the starting protein or starting polypeptide preferably contains as many groups as possible which can be derived and thus acquire an additional negative charge. Preferably, the natural or synthetic oligopeptides, which are used as starting materials, consist to the extent of more than 5%, in particular more than 15% and even more particularly more than 25% of amino acids which contain functional amino groups or other functional basic groups, for example the abovementioned lysine and histidine.

The preparations according to the invention can be administered enterally or parenterally. Preparations which can be administered parenterally or enterally can also be prepared from preparations according to the invention.

Medicinal preparations which contain the negatively charged polypeptides according to the invention can be in the form of powders, suspensions, solutions, sprays, emulsions, ointments or creams and can be used for topical application, for intranasal, rectal or vaginal administration, and also for oral or parenteral (intravenous, intradermal, intramuscular, intrathecal, and the like) administration. Such preparations can be prepared by combining the negatively charged polypeptides (for example by mixing, dissolving or the like) with pharmaceutically acceptable excipients of a neutral type, such as aqueous or non-aqueous solvents, stabilisers, emulsifiers, detergents and additives, and also, if desired, with colorants and aroma substances. The concentration of the negatively charged polypeptide in the preparation according to the invention can vary substantially and be between 0.001, preferably 0.1, and 100%, depending on the mode of administration. Furthermore, the dose of the negatively charged polypeptide to be administered can, for example, be between 0.1 mg and 100 mg/kg of body weight.

The invention also relates to the use of substances which have acquired an additional net negative charge, as defined above, as active substance or as substance contributing to the action, or as carrier for active substance when preparing pharmaceutical preparations which are suitable for treating or curing viral infections and immunodeficiency diseases such as retrovirus infections, including AIDS and AIDS-related diseases, and which can be used to inhibit fusion of infected with non-infected cells.

The invention also relates to a method for treating diseases or disorders caused by retrovirus infections such as AIDS and AIDS-related diseases, or conditions in which fusion of virus with cells or fusion of virus-infected cells with non-infected cells occurs, with which method an additionally negatively charged protein or polypeptide as defined above is used.

The invention also relates to a method for the preparation of modified proteins and polypeptides, with which method the amino groups and/or other basic functional groups of the proteins or polypeptides are derivatised with a reagent which prevents protonation of basic amino acids, the proteins or polypeptides acquiring an additional net negative charge such that the prolongation of the retention time of the protein or polypeptide derivative compared with that of the protein or polypeptide not converted to a derivative is at least 9 minutes, the retention time being determined by means of FPLC (Fast Protein Liquid Chromatography) on an anion exchange column.

The invention also relates to the new modified proteins or polypeptides, comprising a protein or polypeptide in which the amino groups and/or other basic functional groups have been reacted with a reagent which prevents protonation of the basic amino acids and/or other basic groups or replaces said basic amino acids and/or other basic groups by one or more functional groups having a negative charge, the modified protein or polypeptide having an additional net negative charge, compared with the non-modified protein or polypeptide, such that the prolongation of the retention time of the protein or polypeptide derivative, compared with that of the protein or polypeptide not converted to a derivative, is at least 9 minutes, the retention time being determined by means of FPLC on a column containing an anion exchanger.

According to the invention, it has been found that negatively charged polypeptides are, inter alia, powerful and selective anti-HIV agents which substantially reduce syncytium formation and virus-cell fusion, do not bind to the OKT4A epitope of the CD4 receptor and have only a slight inhibitory action on the interaction of anti-gp120 and mAb gp120 and the binding of virus cells at very low concentrations. The negatively charged polypeptides used or prepared according to the invention have no or only a low toxicity.

The invention is illustrated with reference to the investigation described below.

### Investigation

### Preparation of negatively charged albumin with the aid of formaldehyde and succinic anhydride (form-HSA and suc-HSA).

500 mg of HSA were dissolved in 50 ml of 0.2 M Na₂CO₃ (pH 10.0), formaldehyde was added to give a final concentration of 20% by weight, based on the starting material, and the solution formed was stirred in the dark for 72 hours at room temperature. In order to remove insoluble material, the solution was filtered through a filter with 0.2 µm openings, purified on a Sephadex G25 column, washed with distilled water on a PM10 membrane in an Amicon "Stirred Cell Concentrator" and finally lyophilised, form-HSA being obtained.

500 mg of HSA were dissolved in 50 ml of 0.2 M K₂HPO₄ (pH 8.0). 500 mg of solid succinic anhydride were added and the solution was stirred until the succinic anhydride had dissolved. The pH was kept between 8.0 and 8.5 using 6 M sodium hydroxide. Purification was carried out as described for form-HSA. Pure suc-HSA was obtained.

The additional net negative charge of the modified albumin was determined with the aid of a FPLC system (Fast Protein Liquid Chromatography) from Pharmacia, Woerden, the Netherlands, using a mono-Q anion exchange column from Pharmacia. Buffer A was a 0.02 M Tris.HCl buffer (pH 7.4) and buffer B consisted of buffer A plus 1 M NaCl. Elution was carried out at a rate of 0.25 ml/ min, using a gradient of 100% A to 100% B in 30 minutes. The samples of the substance to be investigated were dissolved in an amount of 1 mg/ml in buffer A and in each case 100 µl were injected into the FPLC system.

MT-4 cells from a T₄ lymphocyte cell line carrying HTLV-I were used for the anti-HIV-1 test. The MT-4 cells were cultured in RPMI 1640 medium, supplemented with 10% by volume of heat-inactivated fetal calf serum (FCS) and 20 µg/ml of gentamicin. MOLT-4 cells (clone 8, cf. J.Virol. 57, 1159-1162) were used for the test relating to syncytium formation. The cells were kept in a moist atmosphere of 5% CO₂ in air at a temperature of 37°C. Every 3-4 days cells were centrifuged down and transferred to new culture flasks (2 × 10⁵ cells/ml). The cells were analysed periodically for the presence of mycoplasma: they were always found to be free from mycoplasma.

HIV-1 (strain HTLV-III_{B}) was obtained from the culture fluid of HUT-78 cells infected with HIV-1. The virus titre of the culture fluid was determined in MT-4 cells. The virus was stored at -70°C.

The antiviral activity of the negatively charged polypeptides was determined on the basis of the inhibitory effect on virus-induced cytopathogenicity in MT-4 cells and was recorded with the aid of the 3-(4,5-dimethylthiazol-2-yl)-2,5-diphenyltetrazolium bromide (MTT) method, which is described in J.Virol. Methods 20, 309-321.

Syncytium formation was determined as follows. The modified polypeptides were diluted in RPMI and transferred to the wells of microtitre plates. 5 × 10⁴ HIV-1-infected HUT-78 cells, which had previously been washed twice in order to remove free virus particles, were then added to the wells, immediately followed by the addition of 5 × 10⁴ MOLT-4 cells, a final volume of 200 µl being obtained. The cell mixtures were cultured at 37°C in a CO₂-containing cell incubator. The first syncytia were formed after 4-6 hours. After 24 hours the cells were analysed by microscopic examination and laser flow cytofluorography.

The assay relating to virus adsorption was carried out as follows.

MT-4 cells were exposed to HIV-1 virions in the absence or the presence of the negatively charged polypeptides according to the invention. After incubation for 30 minutes at 37°C, the cells were washed in order to remove non-bound virus particles. The cells were then stained for indirect fluorescence using polyclonal antibody for HIV-1, and the HIV-1 particles bound to the cells were analysed by laser flow cytofluorography.

The CD4 immunofluorescence assay was carried out using the method described in No. 42 Proc.Natl.Acad.Sci. USA 86, 3322-3326. MT-4 cells were incubated for various periods at room temperature in the absence and the presence of a negatively charged polypeptide. The cells were then stained with optimum concentrations of the monoclonal antibodies OKT4A-FITC (orthodiagnostics) or anti-leu3a-PE and "Simultest immuno monitoring" with control material (FITC-labelled IgG₁ and PE-labelled IgG₂) (Becton Dickinson) for 20 minutes at 4°C, washed once in PBS and fixed in 0.5 ml of 0.5% by weight paraformaldehyde in PBS.

The investigations described above showed that a clear correlation exists between the antiviral activity and the negative charge. For example, by simple succinylation of the lysine groups of albumin an anionic compound is obtained which has a very low IC₅₀ (1 µg/ml). None of the negatively charged polypeptides tested was found to be cytotoxic at concentrations of up to 1000 µg/ml.

The Aco-HSA material was prepared analogously to the method described above. The anti-HIV activity of this material is very high: IC₅₀ = 0.0056 µg/ml. This compound is therefore 175 times more active than Suc-HSA and is one of the most active compounds known at present (approximately 25 times more active than AZT).

The negatively charged polypeptides according to the invention inhibit the virus adsorption to only a slight degree, for example in a concentration of 100 µg/ml. For comparison: dextran sulphate inhibited virus adsorption virtually completely at a concentration of 25 µg/ml.

The syncytium formation is appreciably reduced by the negatively charged polypeptides according to the invention, for example by 50% at a concentration of only about 2 µg/ml. In order to obtain a similar inhibition with, for example, dextran sulphate, a concentration of 28 µg/ml is needed, which amount is about 50 times higher than the IC₅₀ thereof in the antiviral assay (0.6 µg/ml).

It is known that OKT4A mAb binds to an epitope of the CD4 molecule, which is responsible for the HIV adsorption and can prevent binding of HIV particles to the cells. Aurintricarboxylic acid (ATA) gives specific interaction with this epitope of the CD4 molecule and was used as positive control. The OKT4A mAb-CD4 interaction was completely inhibited by ATA in a concentration of 25 µg/ml. The negatively charged polypeptides according to the invention (and also dextran sulphate) had no influence on this interaction in concentrations of up to 100 µg/ml, from which it can be concluded that the active substances according to the invention do not bind to this epitope of the CD4 receptor.

Persistently HIV-1-infected HUT-78 cells and a specific anti-gp120 mAb, which recognises the V3 region of gp120 and which plays an important role in the formation of giant cells, were used in the investigation of the direct interaction of the negatively charged polypeptides with the viral gp120. Dextran sulphate inhibited the binding of anti-gp120 mAb to gp120 in a manner which is dependent on the concentration, the amounts involved being said to be equal to the amounts which have an antiviral action. The negatively charged polypeptides according to the invention also inhibit the anti-gp120-mAb-gp120 interaction in a manner which is dependent on the concentration. However, the concentration which is needed for a 50% inhibition is many times (for example 100 times) greater than the IC₅₀, from which it can be concluded that shielding of gp120 is probably not the only mechanism of action of the compounds according to the invention.

The mechanism of action of the compounds according to the invention is based on preventing the fusion of the virus with the cells and with a compound from the fusion of infected cells with non-infected cells. Very probably, the interaction of the compounds with viral fusion proteins (such as GP41 in the case of HIV) is essential here. This mechanism of action has never been described before.

### Procedure for and results of further experiments relating to anti-viral and anticoagulant action

Reference is made to the appended Tables 1, 2, 3 and 4.

The antiviral activity of two substances according to the invention (Aco-HSA and Suc-HSA) against viruses causing immunodeficiency is compared with the activity of dextran sulphate, another polyanionic antiviral compound. Conclusion: substances according to the invention are active against HIV-1, HIV-2, FIV and SIV.

The anti-HIV-1 test has already been described above.

HIV-2 (LAV-2_{ROD}) (obtained from Dr. L. Montagnier, Paris, France) was isolated from the medium supernatant of persistently LAV-2_{ROD}-infected MOLT-4 cells. The anti-HIV-2 test is identical to the anti-HIV-1 test.

FIV-48 and FIV-113 were isolated from peripheral mononuclear blood cells of seropositive wildcats. The anti-FIV test was carried out using the method of Egberink et al. (Proc.Natl.Acad.Sci. USA (1990) 87: 3087-3091).

Mitogen-stimulated cat thymocytes were plated out in 1.6 cm² wells (10⁶ cells per ml) in the presence of various concentrations of the substances to be tested. After incubating for 1 hour at 37°C, FIV was added in an amount which corresponds to 6 × 10⁶ cpm reverse transcriptase (RT) activity. After incubating for 1 hour at 37°C, the medium was replaced by fresh medium containing various concentrations of the substances to be tested. The RT activity in the supernatant was determined after incubating for 4 and 6 days at 37°C (see Table 2).

The antiviral activity of two substances according to the invention (Aco-HSA and Suc-HSA) against DNA viruses is compared with the activity of dextran sulphate, another polyanionic antiviral compound. Conclusion: the substances according to the invention have no activity against the DNA viruses tested, in contrast to dextran sulphate.

Anti-CMV test: human embryonic lung (HEL) fibroblasts were cultured in MEM medium to which 10% fetal calf serum, 1% L-glutamine and 0.3% sodium bicarbonate were added. The cells were infected with 100 PFU (plaque forming units) of human cytomegalovirus (CMV, strain AD169 and Davis strain). At the same time, the substances to be tested were added in various concentrations. The virus plaque formation and the cell death caused by the virus were determined as described by Snoeck et al. (1988) 32, 1839-1844 (see Table 2).

The antiviral activity of two substances according to the invention (Aco-HSA and Suc-HSA) against RNA viruses is compared with the activity of dextran sulphate, another polyanionic antiviral compound. Conclusion: The substances according to the invention have no activity against the RNA viruses tested except for influenza. In contrast, dextran sulphate has an activity only against VSV and Sindbis virus.

The activity of the substances against polio virus, Coxsackie virus, VSV, Sindbis, Semliki Forest virus, reovirus, para-influenza virus, HSV-1, HSV-2 and VMW was determined in the following way:

Confluent cell cultures (Vero cells, HeLa cells and primary rabbit kidney cells) were inoculated with the various viruses to 100 times the CCID₅₀ (50% cell culture infective dose) in the presence of various concentrations of the substances to be tested. After 1 hour the medium was replaced by medium in which only the substances to be tested are still present in various concentrations. The cell death caused by the viruses was determined after 1 to 2 days post-infection for VSV; after 2 days for Coxsackie virus, Semlili Forest virus and polio virus; after 2 to 3 days for HSV-1, HSV-2 and Sindbis virus; and after 6 days for para-influenza virus and reo-virus.

The activity of the substances to be tested against Sendai virus and influenza virus was determined by measuring the fusion of the viral membrane with erythrocyte ghosts and liposomes and with BHK-21 cells or LLC-MK₂D cells. The R₁₅-dequenching method as described by Hoekstra et al. (1984), Biochemistry 23, 5675-5681 and Wildschut et al. in Molecular Mechanisms of Membrane Fusion, (S. Ohki, D. Doyle, T.D. Flanagan, S.W. Hui and E. Mayhew, editors) 1988, Plenum Press, New York, pp. 441-450 was used for this purpose.

To summarise: influenza virus and Sendai virus were labelled with R₁₅. This resulted in a 70% self-quenching of the fluorescence. 35 µl of a concentrated suspension of R₁₅-labelled virus were introduced into a cuvette containing liposomes and erythrocyte ghosts of various cells (in HEPES buffer) and the increase in fluorescence (dequenching) caused by the fusion was measured on-line (excitation 560 nm, emission 590 nm). The same procedure was carried out in the presence of various concentrations of the substances to be tested.

Other polyanionic antiviral compounds such as dextran sulphate and heparin have a serious anticoagulant activity as a significant negative side effect. The substances according to the invention do not have this side effect. Only Aco-HSA has a slight anticoagulant activity at high concentrations of, for example, 100 µg/ml (that is more than 10,000 × the antiviral concentration).

**Table 1**

| Inhibitory effect (IC₅₀ µg/ml) of polyanionic compounds on retroviruses | | | | |
|---|---|---|---|---|
| | HIV-1 | HIV-2 | FIV | SIV |
| Suc-HSA | 0.9 | 78 | < 0.1 | + |
| Aco-HSA | 0.025 | 5.9 | < 0.01 | + |
| DS | 0.6 | 0.08 | ND | ND |
| + = activity determined | | | | |
| ND = not determined | | | | |

**Table 2**

| Inhibitory effect (IC₅₀ µg/ml) of polyanionic compounds on DNA viruses | | | | | |
|---|---|---|---|---|---|
| | HSV-1 | HSV-2 | HSV field TK- | VMW | CMV |
| Suc-HSA | >400 | >400 | ND | ND | >400 |
| Aco-HSA | >400 | >400 | >400 | >400 | >400 |
| DS | 0.7 | 1.0 | 2.0 | 2.0 | 0.2 |

**Table 3**

| Inhibitory effect (IC₅₀ µg/ml) of polyanionic compoun on the replication of RNA viruses | | |
|---|---|---|
| Virus | ACO-HSA | DS |
| Coxsackie | >400 | >400 |
| Polio | >400 | >400 |
| VSV | >400 | 7.0 |
| Sindbis | >400 | 2.0 |
| Semliki Forest | >400 | >400 |
| Reo-virus | >400 | >400 |
| para-influenza | >400 | >400 |
| influenza | 0.8 | >400 |
| Sendai | >400 | >400 |

**Table 4**

| Anticoagulant action of the compounds, determined as APTT (sec) | | | | |
|---|---|---|---|---|
| Conc.(µg/ml) | Dextran sulphate | Suc-HSA | Aco-HSA | Heparin |
| 100 | >400 | 37.7 | 51.1 | >400 |
| 50 | | | 42.0 | |
| 30 | | | 37.8 | |
| 20 | | | 35.1 | |
| 10 | 69.4 | 33.0 | 33.0 | 220 |
| 5 | 49.0 | 37.2 | 36.2 | 140 |
| 3 | 43.6 | 35.8 | 35.9 | 65 |
| 1 | 37.4 | 36.2 | 37.0 | 40.5 |
| 0.1 | 37.6 | 38.0 | 37.8 | 40.3 |
| 0.01 | 38.0 | 39.0 | 36.8 | 38.4 |

The compounds were dissolved in PBS of pH 7.2 and 30 µl were added to 270 µl of plasma.

As control, 30 µl of PBS were added to 270 µl of plasma, which resulted in an APTT value of 38.0 sec.
APTT = activated partial thromboplastin time (seconds)
APTT was determined electromechanically using standard methods.

## Claims

1. Modified protein or polypeptide comprising a protein or polypeptide in which the amino groups and/or other basic functional groups have been reacted with a reagent which replaces the amino and/or other basic functional groups by one or more functional groups, wherein:
(a) the functional groups are selected from cis-aconitate groups, the cis-aconitate groups being derived from cis-aconitic anhydride,
(b) the modified protein or polypeptide has an additional net negative charge such that the prolongation of the retention time of the protein or polypeptide derivative, compared with that of the protein or polypeptide not converted to the derivative, is at least 9 minutes, and
(c) the modified protein or polypeptide contains a polyanionic polypeptide structure.

2. Modified protein or polypeptide according to claim 1, wherein the modified protein or polypeptide have been obtained from polyamino acids and modified plasma proteins.

3. Method for the preparation of modified proteins or polypeptides, wherein the amino groups and/or other basic functional groups of the proteins or polypeptides are derivatised with a reagent which replaces the amino and/or other basic functional groups by one or more functional groups, wherein:
(a) the functional groups are selected from cis-aconitate groups,
(b) the reagent is cis-aconitic anhydride,
(c) the modified protein or polypeptide has an additional net negative charge such that the prolongation of the retention time of the protein or polypeptide derivative, compared with that of the protein or polypeptide not converted to the derivative, is at least 9 minutes, and
(d) the proteins or polypeptides acquire a polyanionic polypeptide structure.

4. Method according to claim 3, wherein the modified protein or polypeptide have been obtained from polyamino acids and modified plasma proteins.

5. Pharmaceutical preparations for treating or curing HIV-infections, which preparations contain modified proteins or polypeptides according to any of claims 1-2.

6. Pharmaceutical preparations according to claim 5, wherein the HIV-infection is a HIV-1 infection.

7. Pharmaceutical preparations for treating or curing influenza, which preparations contain modified proteins or polypeptides according to any of claims 1 - 2.

8. Use of a modified protein or polypeptide according to any of claims 1 - 2 for the preparation of a medicament for the treatment of HIV-infections.

9. Use according to claim 8, wherein the HIV-infection is a HIV-1 infection.

10. Use of a modified protein or polypeptide according to any of claims 1 - 2 for the preparation of a medicament for the treatment of influenza.

## Revendications

1. Protéine ou polypeptide modifié(e) comprenant une protéine ou un polypeptide dans lequel (laquelle) les groupes amines et/ou d'autres groupes fonctionnels basiques ont été mis à réagir avec un réactif qui remplace les groupes amines et/ou les autres groupes fonctionnels basiques par un ou plusieurs groupes fonctionnels, dans lequel (laquelle) :
(a) les groupes fonctionnels sont choisis parmi des groupes cis-aconitate, les groupes cis-aconitate étant dérivés de l'anhydride cis-aconitique,
(b) la protéine ou le polypeptide modifié(e) possède une charge négative nette supplémentaire de sorte que la prolongation de la durée de rétention du dérivé de la protéine ou du polypeptide, par rapport à celle de la protéine ou du polypeptide non converti(e) en dérivé, est d'au moins 9 minutes, et
(c) la protéine ou le polypeptide modifié(e) contient une structure polypeptidique polyanionique.

2. Protéine ou polypeptide modifié(e) selon la revendication 1, dans lequel (laquelle) la protéine ou le polypeptide modifié(e) ont été obtenus à partir d'acides polyaminés et de protéines plasmatiques modifiées.

3. Procédé de préparation de protéines ou de polypeptides modifiés, dans lequel les groupes amines et/ou d'autres groupes fonctionnels basiques des protéines ou des polypeptides sont convertis en dérivé par un réactif qui remplace les groupes amines et/ou les autres groupes fonctionnels basiques par un ou plusieurs groupes fonctionnels, dans lequel :
(a) les groupes fonctionnels sont choisis parmi des groupes cis-aconitate,
(b) le réactif est l'anhydride cis-aconitique,
(c) la protéine ou le polypeptide modifié(e) possède une charge négative nette supplémentaire de sorte que la prolongation de la durée de rétention du dérivé de la protéine ou du polypeptide, par rapport à celle de la protéine ou du polypeptide non converti(e) en dérivé, est d'au moins 9 minutes, et
(d) les protéines ou les polypeptides acquièrent une structure polypeptidique polyanionique.

4. Procédé selon la revendication 3, dans lequel la protéine ou le polypeptide modifié(e) ont été obtenus à partir d'acides polyaminés et de protéines plasmatiques modifiées.

5. Préparations pharmaceutiques destinées au traitement ou à la guérison d'infections par le VIH, lesquelles préparations contiennent des protéines ou des polypeptides modifiés selon l'une des revendications 1 et 2.

6. Préparations pharmaceutiques selon la revendication 5, dans lesquelles l'infection par le VIH est une infection par le VIH-1.

7. Préparations pharmaceutiques destinées au traitement ou à la guérison de la grippe, lesquelles préparations contiennent des protéines ou des polypeptides modifiés selon l'une des revendications 1 et 2.

8. Utilisation d'une protéine ou d'un polypeptide modifié(e) selon l'une des revendications 1 et 2, pour la préparation d'un médicament destiné au traitement d'infections par le VIH.

9. Utilisation selon la revendication 8, dans laquelle l'infection par le VIH est une infection par le VIH-1.

10. Utilisation d'une protéine ou d'un polypeptide modifié(e) selon l'une des revendications 1 et 2, pour la préparation d'un médicament destiné au traitement de la grippe.

## Patentansprüche

1. Modifiziertes Protein oder Polypeptid, umfassend ein Protein oder Polypeptid, worin die Aminogruppen und/oder andere basische funktionelle Gruppen mit einem Reagenz umgesetzt worden sind, welches die Amino- und/oder die anderen basischen funktionellen Gruppen durch ein oder mehrere funktionelle Gruppen ersetzt, worin:
(a) die funktionellen Gruppen ausgewählt sind aus cis-Aconitat-Gruppen, wobei die cis-Aconitat-Gruppen von cis-Aconitsäureanhydrid abgeleitet sind.
(b) das modifizierte Protein oder Polypeptid eine zusätzliche negative Nettoladung aufweist, sodass die Verlängerung der Retentionszeit des Protein- oder Polypeptid-Derivats im Vergleich zu der des Proteins oder Polypeptids, welches nicht in das Derivat umgewandelt wurde, mindestens 9 Minuten beträgt und
(c) das modifizierte Protein oder Polypeptid eine polyanionisiche Polypeptidstruktur enthält.

2. Modifiziertes Protein oder Polypeptid nach Anspruch 1, worin das modifizierte Protein oder Polypeptid aus Polyaminosäuren und modifizierten Plasmaproteinen erhalten worden ist.

3. Verfahren zur Herstellung von modifizierten Proteinen oder Polypeptiden, worin die Aminogruppen und/oder andere basische funktionelle Gruppen der Proteine oder Polypeptide mit einem Reagenz derivatisiert werden, welches die Amino- und/oder die anderen basischen funktionellen Gruppen durch ein oder mehrere funktionellen Gruppen ersetzt, worin:
(a) die fonktionellen Gruppen ausgewählt sind aus cis-Aconitat-Gruppen,
(b) das Reagenz cis-Aconitsäureanhydrid ist,
(c) das modifizierte Protein oder Polypeptid eine zusätzliche negative Nettoladung aufweist, sodass die Verlängerung der Reteutionszeit des Protein- oder Polypeptid-Derivats im Vergleich zu der des Proteins oder Polypeptids, welches nicht in das Derivat umgewandelt wurde, mindestens 9 Minuten beträgt und
(d) die Proteine oder Polypeptide eine polyanionische Polypeptidstruktur erlangen.

4. Verfahren nach Anspruch 3, worin das modifizierte Protein oder Polypeptid aus Polyaminosäuren und modifizierten Plasmaproteinen erhalten worden ist.

5. Pharmazeutische Zubereitungen zur Behandlung oder Heilung von HIV-Infektionen, wobei die Zubereitungen modifizierte Proteine oder Polypeptide nach einem der Ansprüche 1 bis 2 enthalten.

6. Pharmazeutische Zubereitungen nach Anspruch 5, worin die HIV-Infektion eine HIV-1-Infektion ist.

7. Pharmazeutische Zubereitungen zur Behandlung oder Heilung von Grippe, wobei die Zubereitungen modifizierte Proteine oder Polypeptide nach einem der Ansprüche 1 bis 2 enthalten.

8. Verwendung eines modifizierten Proteins oder Polypeptids nach einem der Ansprüche 1 bis 2 für die Herstellung eines Medikaments zur Behandlung von HIV-Infektionen.

9. Verwendung nach Anspruch 8, worin die HIV-Infektion eine HIV-1-Infektion ist.

10. Verwendung eines modifizierten Proteins oder Polypeptids nach einem der Ansprüche 1 bis 2 für die Herstellung eines Medikaments zur Behandlung von Grippe.
